# EUROPÄISCHE PATENTANMELDUNG

(11) **EP 1 148 070 A1**
(43) Veröffentlichungstag der Anmeldung: **24.10.2001**
(21) Anmeldenummer: 01107845.8
(22) Anmeldetag: 09.04.2001
(51) Int. Cl.: C08F 8/12

(54) **Verfahren zur Herstellung von superabsorbierenden Polymeren aus Polyacrylnitrilen**

(30) Priorität: 20.04.2000 DE 10019756
(71) Anmelder: BAYER AG, 51368 Leverkusen (DE)
(72) Erfinder: Michels, Gisbert Dr., 51375 Leverkusen (DE); Gross, Thomas Dr., 42579 Heiligenhaus (DE)

(57) **Zusammenfassung**

Super-absorbierende Polymere (SAP) durch alkalische Hydrolyse von Polyacrylnitril-Emulsionen (PAN-Emulsion), dadurch gekennzeichnet, dass zur Isolierung keine Alkohole oder organische Lösungsmittel eingesetzt werden.

## Beschreibung

Die Erfindung betrifft ein Verfahren zur Herstellung von superabsorbierenden Polymeren (SAP) durch alkalische Hydrolyse von Polyacrylnitril-Emulsionen (PAN-Emulsion).

Superabsorbierende Polymerisate sind bekannt und werden hauptsächlich bei der Herstellung von Windeln und Inkontinenzartikeln, aber auch als wasserspeichernde Materialien in der Landwirtschaft sowie bei der Ummantelung von Elektrokabeln verwendet. In der Regel handelt es sich bei diesen superabsorbierenden Polymerisaten um weitmaschig vernetzte, wasserunlösliche Polymerisate oder Copolymerisate auf Basis von Alkalisalzen der Polyacrylsäure oder Copolymerisate von Akalisalzen der Acrylsäure und Acrylamid, die durch radikalisch initiierte Copolymerisation von Acrylsäure und polyfunktionellen Monomeren, wie Divinylbenzol, Ethylenglykoldimethacrylat, Ethylenglykoldiallylether, Butandioldiacrylat, Hexandioldimethacrylat, Polyglykoldiacrylat, Trimethylolpropan-diacrylat, Allylacrylat, Diallylacrylamid, Trisallylamin, Diallylether, Methylenbisacrylamid und N-Methylolacrylamid, erhalten werden. Aufgrund ihrer Struktur sind solche Polymerisate in der Lage, unter Quellung und Ausbildung von Hydrogelen große Mengen an Wasser und wässrigen Lösungen aufzunehmen und diese auch unter Druck festzuhalten.

Aus DE-A 196 00 163 ist bekannt, dass durch Hydrolyse von feinteiligen PAN-Emulsionen SAP herstellbar sind. Es wird beschrieben, dass superabsorbierende Polymerisate aus PAN-Emulsionen kontinuierlich und reproduzierbar aus konzentrierten Emulsionen hergestellt werden können, wenn man für die alkalische Hydrolyse der PAN-Emulsion selbstreinigende Reaktoren mit für eine Verweilzeit von 0,5 bis 2 Stunden ausreichendem Volumen einsetzt.

Weiterhin wird beschrieben, dass zur Herstellung von superabsorbierenden Polymeren durch Hydrolyse von PAN-Emulsionen besonders solche Geräte geeignet sind, die längere Verweilzeiten bei hinreichend guter Vermischung und gutem Wärmeübergang bei gleichzeitiger Abführung des während der Hydrolyse entstehenden gasförmigen Ammoniaks erlauben. Besonders geeignet sind sogenannte "List-Reaktoren" (Hersteller: Firma List AG, CH-4422 Arisdorf, Schweiz) mit bis zu 16,5 m³ freiem Volumen, die als Einwellen- und Zweiwellengeräte gebaut werden und spezielle Misch- und Abstreiforgane sowohl auf der Welle als auch im Reaktorgehäuse besitzen. Solche Reaktoren können auch hintereinander geschaltet werden, wodurch eine große Flexibilität beim Einsatz erreicht werden kann.

Nach Beendigung der Hydrolyse kann nach DE-A 196 00 163 das Reaktionsgemisch direkt aus dem List-Reaktor in einen mit einem schnelllaufenden Rührer ausgestatteten Fällungsreaktor, der einen niedrigsiedenden Alkohol, z.B. Methanol oder Ethanol, enthält, kontinuierlich eingetragen werden. Dabei fällt das superabsorbierende Polymerisat als feines, leicht filtrierbares Pulver aus. Nach dem Trocknen und Mahlen auf die gewünschte Korngröße liegt dann der fertige Superabsorber vor.

Weiterhin wird beschrieben, dass zur Neutralisation des nicht verbrauchten Alkalihydroxids bzw. zur Einstellung des pH-Wert des Endproduktes eine Säure zugesetzt werden muss, um den für einen Einsatz in Hygieneartikel, z.B. Babywindeln oder Inkontinenzartikel für Erwachsene, erforderlichen pH-Wert zu erhalten, der zwischen 5,5 und 6,5 liegen sollte.

Da bei dem bekannten Verfahren Alkohole zur Isolierung des Produktes eingesetzt werden müssen, muss das nach der Neutralisation entstandene Alkohol-Wasser-Salz-Gemisch entweder entsorgt oder der Alkohol nach Trennung in den Produktionsprozess zurückgeführt werden. Aus Explosionsschutzgründen ist die Trocknung des Wasser und Alkohol enthaltenden SAP nur unter großem sicherheitstechnischen Aufwand möglich ist. Aus ökonomischen und ökologischen Gründen sind andere Verfahren vorzuziehen.

Nach DE-A 196 00 163 soll es alternativ zur Aufarbeitung mit Alkoholen möglich sein, das Hydrolyseprodukt im Vakuum bei Temperaturen bis 100°C zu trocknen. Die Produkte ohne Neutralisation des nicht verbrauchten Alkalihydroxids besitzen dann einen für den Einsatz in Hygieneartikel, z.B. Babywindeln oder Inkontinenzartikel für Erwachsene, zu hohen pH-Wert größer als 7,0.

Der Erfindung lag deshalb die Aufgabe zugrunde, ein SAP mit einem pH-Wert kleiner oder gleich 7,0 durch alkalische Hydrolyse von PAN-Emulsionen oder PAN-Fällungspolymerisaten bereitzustellen, ohne dass Alkohole oder irgendwelche andere organische Lösungsmittel zur Isolierung eingesetzt werden müssen.

Gegenstand der Erfindung ist ein Verfahren zur Herstellung von SAP, das folgende Teilschritte enthält:
a) Herstellung einer PAN-Emulsion (wie oben beschrieben nach DE-A 42 33 026), vorzugsweise unvernetzte oder vernetzte Homopolymerisate des Acrylnitrils,
b) Alkalische Hydrolyse der PAN-Emulsionen in selbstreinigenden, für hochviskose Medien geeigneten Reaktoren,
c) Neutralisation des Hydrolysats:
   1. Zugabe von Säure zum Hydrolyseprodukt oder
   2. Zugabe des Hydrolyseproduktes zu einem Wasser/Säuregemisch zur Einstellung des pH-Wertes,
d) Trocknen des wasserhaltigen, gelförmigen Produktes,
e) Mahlen auf die gewünschte Korngröße
f) Oberflächenmodifikation des kornförmigen SAP

Es wurde gefunden, dass im Gegensatz zu DE-A 196 00 163 zur Isolierung des SAP im erfindungsgemäßen Verfahren kein organisches Lösungsmittel notwendig ist. Weiterhin ist wesentlich, dass dem wasserhaltigen, gelförmigen Produkt Säure zur Neutralisation des Alkalihydroxids und Einstellung des pH-Wertes zugesetzt wird (gemäß c1.)). Im Gegensatz zu DE-A 196 00 163 verbleiben die dabei entstehenden Salze nach der Trocknung im Produkt. Überraschend dabei ist, dass hervorragende Eigenschaften auch in Anwesenheit des aus der Neutralisation stammenden Salzes erhalten werden. Alternativ dazu kann das entstandene Hydrolysat zur Neutralisation auch in ein Gemisch bestehend aus Wasser/Säure gegeben werden (gemäß c2.)) und nach einiger Zeit wieder isoliert werden. Die sich hierbei bildenden niedermolekularen Salze werden ausgewaschen bzw. verbleiben dabei in der Flotte. Die Eigenschaften der durch diesen Prozess resultierenden Produkte sind ebenfalls hervorragend.

Die PAN-Emulsionen nach Teilschritt a) weisen eine durch Laser-Korrelationsspektroskopie ermittelte mittlere Teilchengröße von 60 bis 500 nm, bevorzugt 80 bis 200 nm, und einen Feststoffgehalt von 10 bis 55 Gew.-% auf. Die Molekulargewichte (Gewichtsmittel) der unvernetzten PAN-Emulsionen liegen zwischen 5*10⁵ bis 1*10⁷ g/mol. Bevorzugte PAN-Emulsionen sind unvernetzte oder vernetzte Homopolymerisate des Acrylnitrils. Vernetzte PAN-Emulsionen enthalten einpolymerisierte polyfunktionelle Monomere, wie in DE-A 42 33 026 beschrieben, von 0,2 bis 4,0 Gew.-% bezogen auf die Gesamtmonomermenge.

Nach Teilschritt b) werden die dünnflüssigen Edukte (PAN-Emulsion und wässrige Alkalihydroxidlösung) gemischt und die Hydrolyse in selbstreinigenden, für hochviskose Medien geeignete Reaktoren (HV-Reaktor) durchgeführt. Bei der Hydrolyse entstehen hochviskose Gele mit einem Feststoffgehalt von 10 bis 70 Gew.-%. Geeignete HV-Reaktoren besitzen eine gute Misch- und Knetwirkung für das entstehende hochviskose Gel, einen guten Wärmeübergang und erlauben eine Abrührung des bei der Hydrolyse entstehenden Ammoniaks. Besonders geeignete HV-Reaktoren sind die der Fa. List AG, CH-4422 Arisdorf, Schweiz, die als Ein- und Zweiwellengeräte gebaut werden und Misch- und Abstreiforgane sowohl auf der Welle als auch im Reaktorgehäuse besitzen.

Die Hydrolyse kann nach Vermischen der Edukte im Batchverfahren, im semi-kontinuierlichen Verfahren durch Dosierung eines oder beider Edukte oder im kontinuierlichen Verfahren hergestellt werden. Auch bei einem kontinuierlichen Verfahren ist es möglich, die Edukte zu Beginn des Prozesses oder teilweise später im Prozess zu dosieren. Es ist möglich einen oder mehrere hintereinander geschaltete HV-Reaktoren einzusetzen. Die Vermischung der Edukte kann im HV-Reaktor oder vorgeschalteten Apparaten, wie z.B. Rohrleitungen, Statikmischern, aber auch einem vorgeschalteten HV-Reaktor, der überwiegend Mischaufgaben hat, oder in Schnecken-Knet-Maschinen durchgeführt werden.

Bei der Hydrolyse der PAN-Emulsionen mit wässrigen Alkalihydroxidlösungen beträgt das molare Verhältnis der Nitrilgruppen des Polymeren zum Alkalihydroxid 1:1 bis 1:0,05, bevorzugt 1:0,9 bis 1:0,1, besonders bevorzugt 1:0,7 bis 1:0,3. Alkalihydroxide sind bevorzugt NaOH oder KOH oder deren Mischungen. Die Hydrolyse wird bei 80 bis 200°C, bevorzugt über 100 bis 160°C unter Überdruck ausgeführt. Die Reaktionszeiten betragen 0,01 bis 10 Stunden. Das bei der Hydrolyse entstehende gasförmige Ammoniak wird aus dem Reaktor entfernt und in Wasser gelöst oder bei tiefen Temperaturen kondensiert. Zur möglichst vollständigen Entfernung des Ammoniaks aus dem Hydrolyseprodukt ist es günstig, neben Ammoniak zusätzlich Wasser aus dem Reaktor zu verdampfen. Der dadurch entstandene Verlust an Wasser kann durch Zudosieren von Wasser (flüssig oder dampfförmig) kompensiert werden. Es ist aber auch möglich den entstandenen Verlust nicht zu kompensieren, so dass der Feststoffgehalt des Hydrolyseproduktes entsprechend steigt.

Bei der Hydrolyse entstehen teilhydrolysierte Polymere, bei denen 30 bis 80 Mol-% der Nitrilgruppen des PAN-Polymers in Carboxylatgruppen und 20 bis 70 Mol-% in Carbonamidgruppen umgewandelt sind und 0 bis 20 Mol-% der Nitrilgruppen unverändert bleiben. Der pH-Wert der Hydrolyseprodukte, gemessen als 0,1 gew.-%ige Lösung in 0,9 gew.-%iger NaCl-Lösung liegt im Bereich von 8,0, bevorzugt von 7,5 bis 7,1.

Nach Teilschritt c) wird zur Einstellung des pH-Wertes auf einen Bereich von 7,0, bevorzugt 6,5 bis 5,5, gemessen als 0,1 gew.-%ige Lösung in 0,9 gew.-%iger NaCl-Lösung, dem Hydrolyseprodukt nach beendeter Hydrolyse eine Säure zugesetzt (gemäß c1.)) bzw. das Hydrolyseprodukt kurzzeitig in ein Wasser/Säuregemisch geleitet (gemäß c2.)).

Geeignete Säuren nach c1.) sind Mineralsäuren, wie z.B. Salzsäure, Schwefelsäure, Phosphorsäure, Salpetersäure, Carbonsäuren, auch mehrwertige Säuren und Säuren mit weiteren funktionellen Gruppen, z.B. Ameisensäure, Essigsäure, Propionsäure, Adipinsäure, Milchsäure, Zitronensäure, polymere Säuren, z.B. Polyacrylsäure, Polyacrylsäure-copolymerisate, Polystyrolsulfonsäure sowie Mischungen der vorgenannten Säuren. Polymere Säuren können in vernetzter oder unvernetzter Form eingesetzt werden.

Die Menge an zugesetzter Säure beim Teilschritt c1.) richtet sich nach dem für die Anwendung gewünschten pH-Wert einerseits und der Säurestärke der Säure andererseits. Die Menge an zugesetzter Säure beträgt deshalb 0,1 bis 20 Gew.-%, bevorzugt 0,1 bis 10 Gew.-% bezogen auf das Trockengewicht des Hydrolyseproduktes. Die durch die Zugabe der Säure entstehenden Alkalisalze verbleiben im Produkt. Die Säure kann mit dem hochviskosen Hydrolyseprodukt in geeigneten Mischern, wie z.B. Statikmischern, Schnecken-Knetmaschinen, Knetern, kontinuierlich oder diskontinuierlich vermischt werden.

Geeignete Säuren nach c2.) sind Mineralsäuren, wie z.B. Salzsäure, Schwefelsäure, Phosphorsäure, Salpetersäure, Carbonsäuren, auch mehrwertige Säuren und Säuren mit weiteren funktionellen Gruppen, z.B. Ameisensäure, Essigsäure, Propionsäure, Adipinsäure, Milchsäure, Zitronensäure, polymere Säuren, z.B. Polyacrylsäure, Polyacrylsäure-copolymerisate, Polystyrolsulfonsäure sowie Mischungen der vorgenannten Säuren. Polymere Säuren können in vernetzter oder unvernetzter Form eingesetzt werden.

Die Menge an zugesetzter Säure beim Teilschritt c2.) richtet sich nach dem für die Anwendung gewünschten pH-Wert einerseits und der Säurestärke der Säure andererseits. Die Menge an zugesetzter Säure beträgt deshalb 0,1 bis 20 Gew.-%, bevorzugt 0,1 bis 10 Gew.-% bezogen auf das Trockengewicht des Hydrolyseproduktes. Die Säure wird in der 1,0 bis 100fachen Menge an Wasser, bevorzugt 1,5 bis 30fachen Menge an Wasser bezogen auf das Gewicht des zu neutralisierenden SAP-Gels gelöst. Die Verweilzeit des Hydrolysats in dem Wasser/Säuregemisch liegt zwischen 5s und 30min, bevorzugt zwischen 10s und 5min. Die Neutralisation des Hydrolyseproduktes nach Teilschritt c2.) kann sowohl batchweise als auch kontinuierlich durchgeführt werden.

Das nach Teilschritt c) erhaltene wasserhaltige, gelförmige Produkt wird nach Teilschritt d) getrocknet. Geeignete Trocknungsverfahren sind nach dem Stand der Technik beschrieben in "Modern Superabsorbent Polymer Technology", Hrsg. F.L. Buchholz, A.T. Graham, Wiley-VCH, New York, 1998, ISBN 0-471-19411-5, Kapitel 3.2.4.2. Post-Reactor Gel Preperation, S. 85-87 und 3.2.5. Drying, S. 87-93 und der dort zitierten Literatur. Nach der Trocknung verbleiben im Gegensatz zu DE 196 00 163 A1 die durch Zugabe einer Säure nach Teilschritt c1.) entstehenden Salze im Produkt.

In Teilschritt e) wird das getrocknete Produkt nach dem Stand der Technik gemahlen und klassiert; siehe hierzu: "Modern Superabsorbent Polymer Technology", Hrsg. F.L. Buchholz, A.T. Graham, Wiley-VCH, New York, 1998, ISBN 0-471-19411-5, Kapitel 3.2.6. Handling of the Dried Material: Particle Sizing, S. 93-95. Die Teilchengrößenverteilung des gemahlenen und klassierten SAP soll im Bereich von 100 bis 1000 µm, bevorzugt 150 bis 850 µm liegen.

Zur Verbesserung der Absorptionseigenschaften, insbesondere zur Vermeidung von Gel-blocking, das beim Einsatz der SAP in Hygieneartikeln, wie z.B. Babywindeln, zu einer niedrigen Anfangstransportgeschwindigkeit der aufzunehmenden Flüssigkeit und einem schlechten Flüssigkeitstransport führt, und zu einer Verbesserung der Absorbancy Under Load (AUL), d.h. einer Absorption unter hoher Druckbelastung von bspw. 0,7 psi, muss eine Oberflächenvernetzung bzw. -modifizierung der kornförmigen SAP durchgeführt werden. Wesentlich dabei ist, dass im Teilschritt f) weitere Säure zur Einstellung des pH-Wertes zugesetzt werden kann. Es kann dabei die gleiche Säure wie im Teilschritt c) oder eine andere Säure aus der o.g. Gruppe der Säuren eingesetzt werden. Die Säure wird in Mengen von 0 bis 10 Gew.-%, bevorzugt 0 bis 6 Gew.-% bezogen auf SAP zusammen mit dem Mittel zur Oberflächenvernetzung eingesetzt.

Bevorzugte Oberflächenmodifizierungsmittel bei Einsatz von Säure sind Di- und Polyole, z.B. Ethylenglykol, 1,2-Propandiol, Glycerin, Trimethylolpropan. Diese Modifizierungsmittel werden gemeinsam mit der Säure in Lösung, die üblicherweise aus einer Alkohol-Wasser-Mischung besteht, auf die SAP-Partikel aufgesprüht und vermischt und innerhalb 0,1 bis 5, bevorzugt 0,5 bis 3 Stunden bei 100 bis 200°C, bevorzugt 110 bis 180°C zur Reaktion gebracht. Es ist aber auch möglich die o.g. Oberflächenmodifizierungsmittel ohne Säure einzusetzen. Weiterhin eignen sich Ethylencarbonat, Diglycidylverbindungen, wie z.B. Ethylenglykoldiglycidylether oder Di- oder Polyisocyanate, gegebenenfalls Mischungen der genannten Mittel.

Die nach dem erfindungsgemäßen Verfahren erhältlichen SAP eignen sich hervorragend für den Einsatz bei der Herstellung von Hygieneartikeln, wie z.B. Babywindeln, Inkontinenz-artikeln für Erwachsene und Frauenhygiene, zur Ummantelung von Elektrokabeln, für den Einsatz als wasserspeichernde Materialien in der Landwirtschaft und für den Verpackungsbereich von auslaufgefährdeten Gütern.

Zur Charakterisierung der Produkte wird die Absorption (gemäß EDANA 440.0-96), Retention (gemäß EDANA 441.0-96), AUL bei 0,3 und 0,7 psi (gemäß EDANA 442.0-96) und der pH-Wert (0,1 gew.-%ige Lösung in 0,9 gew.-%iger NaCl-Lösung) gemessen.

### Beispiele 1-3: Hydrolyse

Die alkalischen Hydrolysen der PAN-Emulsionen wurden in einem kontinuierlichen Verfahren, bestehend aus einem Zweiwellenextruder (Reaktionsvolumen: 0,5 L) und einem HV-Reaktor der Fa. List (List DTB 6 Conti, 17,5 L Reaktionsvolumen) durchgeführt.

Die PAN-Emulsion wurde nach DE 42 33 026 A1, Bsp. 8 hergestellt. Es handelt sich hierbei um ein unvernetztes Polyacrylnitrilhomopolymerisat, der Anteil des eingesetzten polymeren Emulgators betrug im Gegensatz zu DE 42 33 026 A1, Bsp. 8 nur 2,5 Gew.-% bezogen auf Polyacrylnitril.

Die Edukte (PAN-Emulsion, Natronlauge, E-Wasser) wurden in den Zweiwellenextruder gepumpt (Dosierflüsse, Drehzahl und Heiztemperatur des Extruders: s. Tab. 1). Das viskose Produkt, das den Extruder verließ, wurde in den HV-Reaktor dosiert. Nach Durchlaufen des HV-Reaktors wurde das viskose, gelartige, schwach gelbe bis farblose Produkt von der Austragsschnecke über eine Viellochdüse ausgetragen (Heiztemperatur, Drehzahl der Hauptwelle des HV-Reaktors und der Austragsschnecke: s. Tab. 1). Das entstandene Ammoniak-Wasser-Gemisch wird aus dem HV-Reaktor über eine Druckhaltung in einen Säurewäscher entspannt.

### Beispiele 1a, 2a: Neutralisation im Teilschritt c1.)

In Tab. 2 sind die Säuren, die im Teilschritt c1.) (Beispiel 1a, 2a) zur Einstellung des pH-Wertes benutzt wurden, und die Mengen in Gew.-% bezogen auf den Feststoff des gelförmigen SAP angegeben. Nach Zugabe der Säure und Vermischen in einem Kneter wird das Gel in einem Labortrockenschrank bei 110-115°C getrocknet, mit einer Labormühle gemahlen und auf eine Teilchengröße von 100-800 µm fraktioniert.

Die Oberflächenmodifikation wurde mit einer Lösung aus Ethylenglykoldiglycidylether, 1,2-Propandiol und Wasser im Verhältnis 0,06:3,152:0,788 durchgeführt. Hierzu wurden in einem Becherglas 100 Tle. SAP-Pulver vorgelegt, mit 4 Tle. der o.g. Lösung besprüht und vermischt und anschließend für 2h bei 125°C in einem Trockenschrank zur Reaktion gebracht (s. Tab.2).

### Beispiele 1b-3b: Oberflächenmodifikation im Teilschritt f)

In Tab. 3 sind die Säuren, die im Teilschritt c1.) und im Teilschritt f) zur Einstellung des pH-Wertes und/oder als Katalysator für die Oberflächenmodifikation benutzt wurden, und die Mengen in Gew.-% bezogen auf den Feststoff des gelförmigen SAP angegeben. Nach Zugabe und Vermischen der Säure im Teilschritt c) wird das Gel in einem Labortrockenschrank bei 110-115°C getrocknet, mit einer Labormühle gemahlen und auf eine Teilchengröße von 100-800 µm fraktioniert.

Zudem ist in Tab. 3 ein Beispiel aufgeführt bei dem die Neutralisation gemäß Teilschritt c2.) durchgeführt wurde (Beispiel 3b). Das feuchte SAP-Gel wurde in der fünffachen Menge Wasser suspendiert, mit der in Tab. 3 angegebenen Menge in Gew.-% bezogen auf den Feststoff des gelförmigen SAP an 85%iger Ameisensäure versetzt und nach 60s isoliert. Das erhaltenen Gel wurde wie oben beschrieben weiter aufgearbeitet.

Die Oberflächenmodifikation wird mit einer Lösung aus Säure, wie in Tab. 3 angegeben, Glycerin, 2-Propanol, Wasser im Verhältnis 1:2:2:2 durchgeführt. Hierzu werden in einem Becherglas 100 Tle. SAP-Pulver vorgelegt, mit 7 Tle. der o.g. Lösung besprüht und vermischt und anschließend für 2h bei 160°C in einem Trockenschrank zur Reaktion gebracht.

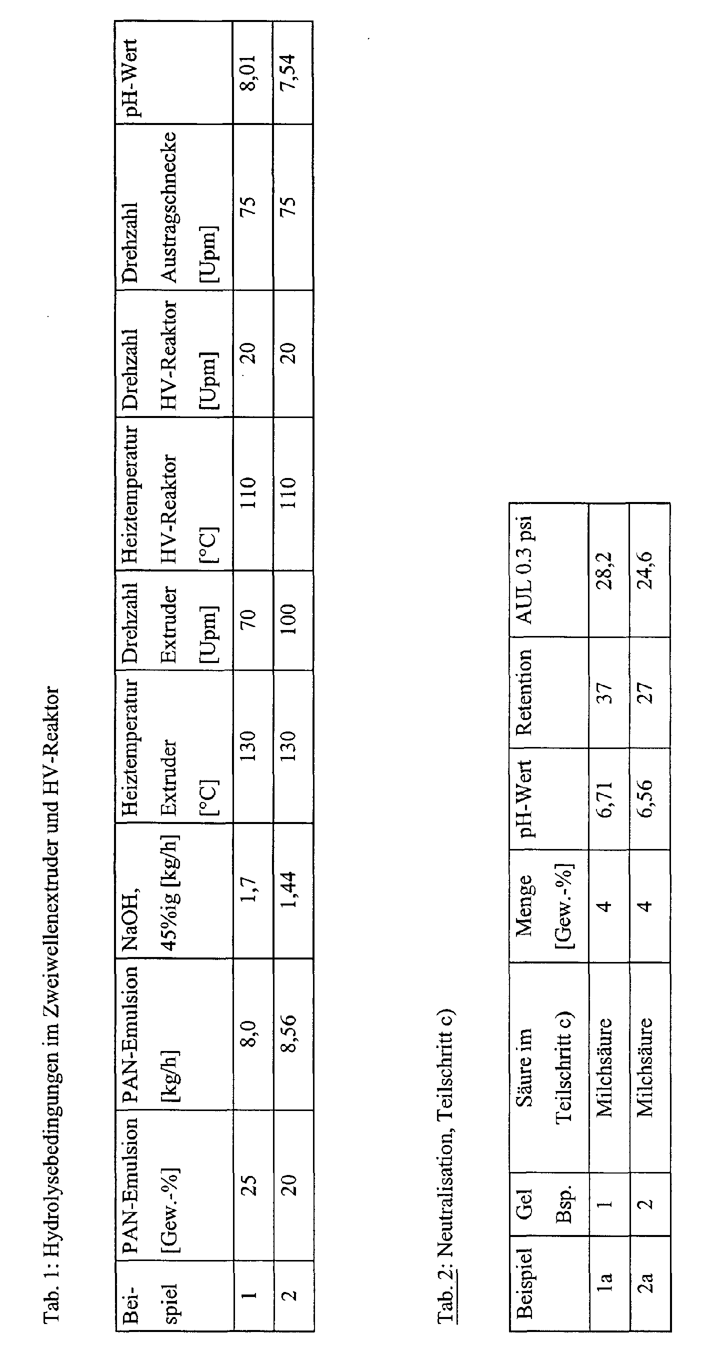

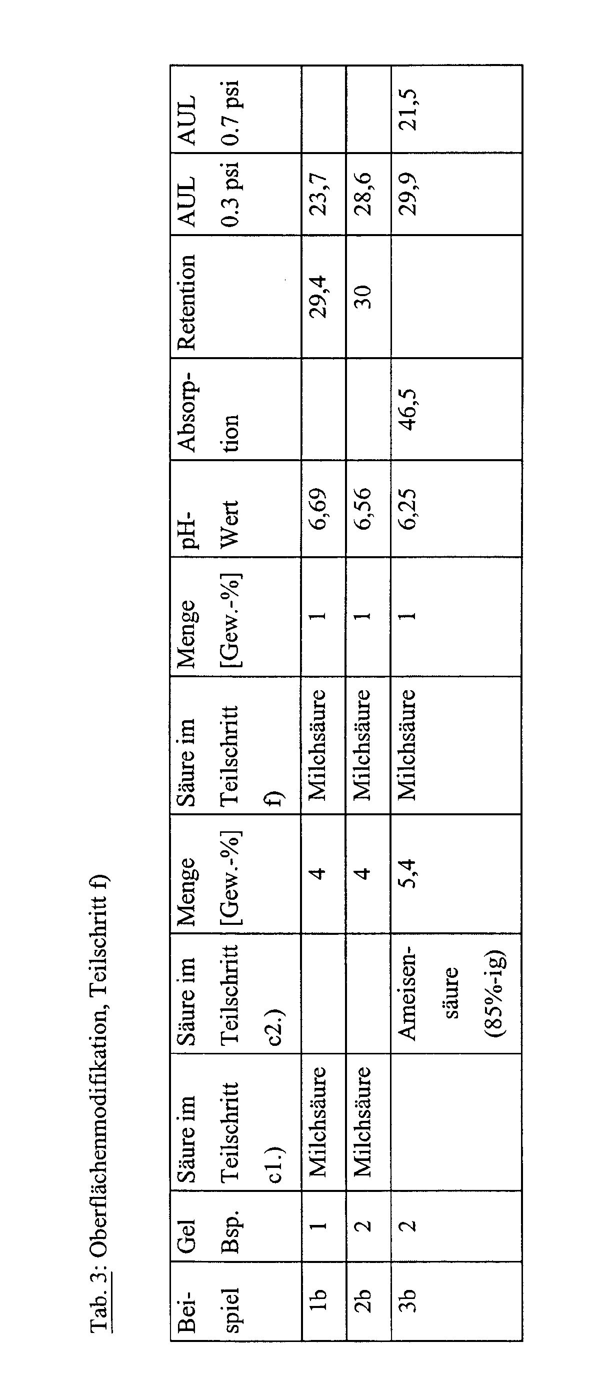

## Patentansprüche

1. Verfahren zur Herstellung von Superabsorbern durch alkalische Hydrolyse von Polyacrylnitril-Emulsionen, **dadurch gekennzeichnet, dass** zur Isolierung keine Alkohole oder organische Lösungsmittel eingesetzt werden.

2. Verfahren gemäß Anspruch 1, wobei die Teilschritte Herstellung einer PAN-Emulsion und alkalische Hydrolyse der Polyacrylnitril-Emulsionen in selbstreinigenden, für hochviskose Medien geeigneten Reaktoren und Neutralisation des Hydrolysats enthalten sind.

3. Verfahren gemäß Anspruch 2, wobei zusätzlich die Verfahrensschritte
d) Trocknen des Produktes und
e) Mahlen
enthalten sind.

4. Verfahren gemäß einem oder mehreren der vorangegangenen Ansprüche, wobei zusätzlich ein Verfahrensschritt
f) Oberflächenmodifikation des Superabsorbers
enthalten ist.

5. Verfahren gemäß einem oder mehreren der vorangegangenen Ansprüche, wobei die Neutralisation des Hydrolysats (Verfahrensschritt c)) durch
1. Zugabe von Säure zum Hydrolyseprodukt oder/und
2. Zugabe des Hydrolyseproduktes zu einem Wasser/Säuregemisch zur Einstellung des pH-Wertes oder/und
durchgeführt wird.

6. Verfahren gemäß einem oder mehreren der vorangegangenen Ansprüche, wobei als Polyacrylnitril-Emulsion unvernetzte oder vernetzte Homopolymerisate des Acylnitrils bei der alkalischen Hydrolyse eingesetzt werden.

7. Verfahren gemäß einem oder mehreren der vorangegangenen Ansprüche, wobei die PAN-Emulsion (in Teilschritt a)) eine mittlere Teilchengröße von 60 bis 500 nm aufweist.

8. Verfahren gemäß einem oder mehreren der vorangegangenen Ansprüche, wobei die Molekulargewichte der unvernetzten PAN-Emulsionen zwischen 5·10⁵ und 1·10⁷ g/mol liegen.

9. Verfahren gemäß einem oder mehreren der vorangegangenen Ansprüche, wobei in Verfahrensschritt b) hochviskose Gele mit einem Feststoffgehalt von 10 bis 70 Gew.-% entstehen.

10. Verfahren gemäß einem oder mehreren der vorangegangenen Ansprüche, wobei im Verfahrensschritt e) die Teilchengrößenverteilung der gemahlenen und klassierten Superabsorper im Bereich von 100 bis 1000 µm liegt.

11. Superabsorber, hergestellt gemäß einem oder mehrerer der vorangegangenen Ansprüche.

12. Verwendung der Superabsorber gemäß Anspruch 11 zur Herstellung von Windeln, Inkontinenzartikeln, wasserspeichernden Materialien und Elektrokabelummantelungen.

13. Windeln, Inkontinenzartikeln, wasserspeichernden Materialien und Elektrokabelummantelungen, hergestellt gemäß einem oder mehreren der vorangegangenen Ansprüche.
